# EUROPEAN PATENT APPLICATION

(11) **EP 4 782 026 A1**
(43) Date of publication of application: **29.07.2026**
(21) Application number: 25154112.4
(22) Date of filing: 27.01.2025
(51) Int. Cl.: A61M 1/36, G01L 19/00

(54) **TRANSDUCER PROTECTOR FOR AN EXTRACORPOREAL CIRCUIT**

(71) Applicant: Fresenius Medical Care Deutschland GmbH, 61352 Bad Homburg (DE); Fresenius Medical Care AG, 61352 Bad Homburg (DE)
(72) Inventor: CAVALLI, Riccardo, 61352 Bad Homburg (DE); VENERONI, Alain, 61352 Bad Homburg (DE); BRONZATO, Luca, 61352 Bad Homburg (DE); BREUNINGER, Marcus, 61352 Bad Homburg (DE)
(74) Representative: Lorenz Seidler Gossel Part. mbB

(57) **Abstract**

The present invention comprises a transducer protector for an extracorporeal circuit, comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic membrane arranged in the fluid path for separating the first end of the fluid path from the second end; wherein the second connector is a bayonet connector.

## Description

The present invention relates to a transducer protector for protecting a pressure transducer connected to an extracorporeal blood circuit.

In medical treatments which require an extracorporeal circuit, in particular in blood-treatments such as hemodialysis, the pressure in the extracorporeal circuit, for example the arterial and/or the venous pressure, must be constantly monitored. This is usually achieved by means of a pressure transducer connected to the main circuit of the extracorporeal circuit by a branch pipe.

In order to protect the pressure transducer, which is typically located inside a dialysis machine, from any contact with the patient's blood, a transducer protector is usually provided between the pipe and the pressure transducer. In particular, the transducer protector may be provided as a part of the extracorporeal circuit as a disposable, and during treatment being connected to a pressure transducer interface.

Document EP 2 226 087 A1 shows a well-established configuration of a transducer protector for an extracorporeal circuit, comprising a main body comprising a fluid path, a first connector for fluidly coupling a first end of the fluid path to a pipe of the extracorporeal circuit, a second connector for fluidly coupling a second end of the fluid path to a pressure transducer interface, and a hydrophobic gas-permeable membrane arranged in the fluid path for separating the first end of the fluid path from the second end.

In this known transducer protector, the second connector is configured as a female type Luer-type connector, comprising a screw section for screwing the second connector onto a corresponding screw section of the pressure transducer interface. Further, the main body of the transducer protector is formed by means of two plastic half-shells enclosing between them a hydrophobic membrane, the two sections forming a circular section arranged between the two tubular connectors. The circular section enclosing the membrane is used as a gripping section for turning the transducer protector for closing and opening the Luer connection.

This transducer protector provides a robust design used for many years in the market. However, it has non-optimal usability and is in particular difficult to handle due to the reduced surface of the circular central element. Further, due to the rotating movement needed to screw the component on the Luer lock male connector, fixation is difficult. Finally, the user is not aware if the transducer protector is properly connected or not, and therefore not aware if the connection is tight. A gas-tight connection between the pressure transducer and the tubing set is essential for a reliable pressure measurement. Further, manufacture of the transducer protector is difficult.

Despite these disadvantages, the standard configuration using two half shells and a Luer lock connection has nevertheless been the generally accepted solution for connecting a transducer protector to the corresponding pressure transducer interface in the market. So far, most efforts in improving known transducer protectors have centered on the improvement of the safety of the separation function of the membrane. For example, document EP 2 408 490 B1 shows a transducer protector using two membranes arranged in series.

The object of the present invention is to provide an improved transducer protector for an extracorporeal circuit and a corresponding pressure transducer interface. In particular, the present invention sets out to avoid at least some of the disadvantages of the known solution described above.

This object is solved by a transducer protector according to claim 1 as well as a pressure transducer interface according to claim 5.

The present invention also relates to systems comprising a transducer protector adapter according to claims 10 and 11 for connecting a transducer protector with a different connector type such as a male or female Luer or screw type connector to a pressure transducer interface according to claim 5 or for connecting a transducer protector according to claim 1 to a pressure transducer interface with a different connector type such as a male or female Luer or screw type connector.

In a first aspect, the present application comprises a transducer protector for protecting a pressure transducer connected to an extracorporeal circuit, the transducer protector comprising: a main body comprising a fluid path; a first connector for fluidly coupling a first end of the fluid path to a pipe of the extracorporeal circuit; a second connector for fluidly coupling a second end of the fluid path to a pressure transducer interface; and a hydrophobic gas-permeable membrane arranged in the fluid path for separating the first end of the fluid path from the second end. In particular, the first connector and the second connector are arranged at the ends of the fluid path extending through the main body of the transducer protector, with the hydrophobic gas-permeable membrane being arranged in the fluid path such that the pipe of the extracorporeal circuit and the pressure transducer interface, when coupled with the transducer protector, are fluidly connected to each other through the hydrophobic gas-permeable membrane. According to the first aspect of the present invention, the second connector is a bayonet connector.

The inventors of the present invention have realized that a bayonet connector is decisively easier to handle than the Luer connectors used in the prior art. Further, the user is provided with a tactile and/or audible feedback, such that proper connection is ensured. A bayonet connector further allows for additional advantages as described in the following.

In an embodiment of the present invention, the second connector is configured to be bayonet engaged with the pressure transducer interface with anaxial movement along the direction of the fluid path at its second end and a defined rotational movement to engage the bayonet connection.

Because the bayonet connection provided by the second connector is engaged with an axial movement along the direction of the fluid path at its second end and a defined rotational movement to engage the bayonet connection, establishing the connection with the pressure transducer interface is simpler than in prior art solutions requiring a rotating screw-like movement with rising torque. Thereby, the usability is improved.

In an embodiment of the present invention, the first connector is a tubular element configured for receiving an end section of the pipe of the extracorporeal circuit.

In an embodiment of the present invention, the end section of the pipe is glued into the tubular element.

In an embodiment of the present invention, the first connector comprises a receiving section for a third connector. The third connector may, e.g., be a male or female Luer connector, a bayonet connector or screw-type connector.

In an embodiment of the present invention, the second connector comprises at least one bayonet connecting element, in particular a bayonet connecting element of the peg or pin type.

In an embodiment of the present invention, the second connector comprises a tubular section and at least two bayonet connection geometries of the peg or pin type. The bayonet pegs or pins may be arranged on opposite sides of the tubular section.

In an embodiment of the present invention, the transducer protector comprises a male bayonet connector with at least one radial pin, and a female receptor with at least one matching shaped slot.

In an embodiment of the present invention, the transducer protector comprises a male bayonet element connector with at least two radial pins, and a female receptor with at least two matching slots.

The male bayonet connector may in particular have a cylindrical shape and/or the female receptor may have a cylindrical opening.

In an embodiment of the present invention, the bayonet element is connected to a main body of the transducer protector and has a free end comprising a bayonet geometry for engaging a bayonet mount of the pressure transducer interface, wherein the bayonet geometry in particular comprises at least one pin extending in a radial direction.

In an embodiment of the present invention, the bayonet connection provided by the second connector is a releasable connection.

In an embodiment of the present invention, the second connector comprises at least two separate bayonet elements and/or bayonet connection geometries. The bayonet elements and/or bayonet connection geometries may each be configured as described above for the at least one bayonet element.

In an embodiment of the present invention, the bayonet elements and/or bayonet connection geometries are arranged on opposite sides of an axis defined by the fluid path at its second end. In other words, the two bayonet elements and/or bayonet connection geometries are arranged on 12, and 6 o'clock on a clock face viewed along the fluid path.

In an embodiment of the present invention, the bayonet connector comprises three bayonet connection elements and/or bayonet connection geometries. The three bayonet connection elements and/or bayonet connection geometries may be arranged at equal angular distances of 120°, i.e., 12, 4, and 8 o'clock on a clock face viewed along the fluid path.

In an embodiment of the present invention, the bayonet connector comprises two or more bayonet connection elements and/or bayonet connection geometries which are arranged at unequal or non-uniform angular distances. This may prevent the connection of other bayonet connectors and/or bayonet connection geometries to the transducer interface and, thereby, increase overall safety.

In an embodiment of the present invention, the bayonet connector comprises two or more bayonet connection elements and/or bayonet connection geometries having different width or size or length. This may prevent the connection of other bayonet connectors and/or bayonet connection geometries to the transducer interface and, thereby, increase overall safety.

In an embodiment of the present invention, the second end of the fluid path is configured as a tubular element to be sealingly engaged with a fluid path of the pressure transducer interface, wherein preferably a bayonet connection element or bayonet connection elements of the second connector extend from a connection point with the main body of the transducer protector towards the pressure transducer interface on an outside and with a distance to the tubular element. This allows a reliable sealing provided by the tubular element combined with a reliable bayonet connection provided by the bayonet connection element or bayonet connection elements without any interference of the one with the other.

In a variant, the second end of the fluid path is surrounded by a flange surface laminated with a sealing layer for sealingly engaging the pressure transducer interface, wherein preferably, the hydrophobic gas-permeable membrane is bonded to the sealing layer.

The bonding of the hydrophobic gas-permeable membrane to the body of the transducer protector and/or the sealing layer is preferably done by heat bonding, ultrasonic welding or gluing. The preferred method for bonding the membrane to the sealing layer is ultrasonic welding.

In an embodiment of the present invention, a bayonet geometry of the second connector is configured to engage a counter element of the pressure transducer interface from an inner side. In particular, the bayonet geometry may therefore be provided on the bayonet connection element or elements of the transducer protector on an outer side.

In a second variant, a bayonet geometry of the second connector is configured to engage a counter element of the pressure transducer interface from an outer side.

In an embodiment of the present invention, a handling section of the transducer protector is arranged on a first tubular element of the transducer protector forming the first end of the fluid path of the pressure transducer. The handling section may in particular be provided as two handling elements extending radially from the first tubular element.

The handling section and in particular the handling elements may be connected axially to a ring element of the transducer protector.

In an embodiment of the present invention, the bayonet connection element is connected to the ring element of the transducer protector and a free end of the bayonet connection element extends from the ring element in a direction towards the pressure transducer interface.

In an embodiment of the present invention, the second tubular element forming the second end of the fluid path is connected to and/or extends from the ring section towards the pressure transducer interface.

In an embodiment of the present invention, an outer rim of the membrane is connected to a seat provided on the ring section.

In an embodiment of the present invention, a bayonet element or a bayonet geometry of the second connector and/or a bayonet seat of the pressure transducer interface has a rounded shape, preferably an essentially circular shape with respect to an axis defined by the fluid path at its second end. This allows for a particularly efficient connection. In particular, the shape may be circular.

In an embodiment of the present invention, in a cross section, an outer and/or an inner edge of the bayonet element or a bayonet geometry of the second connector and/or of the bayonet seat of the pressure transducer interface has a rounded shape, preferably an essentially circular shape. In particular, the shape may be circular.

In a first variant, the transducer protector is formed by two molded parts, each part comprising one of the first and the second end of the fluid path, and/or wherein the hydrophobic gas-permeable membrane is arranged between the two molded parts.

In an embodiment, the first part comprises the first connector and the bayonet connection element or elements.

In an embodiment, the second part comprises a second tubular element for connecting the fluid path to the pressure transducer interface. Preferably, the inner diameter of the second tubular element is larger than a diameter of the membrane and/or comprises a bottom section protecting the membrane, the bottom section having a hole.

In an embodiment, the second tubular element extends from an outer edge of the bottom section towards the second side and in particular has, together with the bottom section, a pot shape.

In a second variant of the present invention, the transducer protector is formed by an integrally molded part comprising the entire fluid path and preferably both the first and the second connector. This reduces the production costs.

In a third variant of the present invention, which may be combined with the second variant, the hydrophobic gas-permeable membrane is bonded to a main body of the transducer protector from the side of the second end of the fluid path. In particular, the hydrophobic gas-permeable membrane is bonded to a step portion provided within the fluid path of the transducer protector facing the second end.

In an embodiment of the present invention, the integrally molded part comprises the entire fluid path and at least the sealing sections of the first and the second connector configured for sealingly connecting to the pipe and the pressure transducer interface.

In an embodiment of the present invention, the integrally molded part comprises the entire fluid path, with the second end of the fluid path being formed by a tubular element configured for sealingly connecting to the pressure transducer interface, and in particular to a sealing element of the pressure transducer interface.

In first variant, mechanical locking elements, in particular bayonet elements, of the second connector may be provided as a separate part connected to the integrally molded part.

In second, preferred variant, the integrally molded part comprises the first and the second connector, i.e. all the parts of the first and the second connector.

In an embodiment of the present invention, the integrally molded part transducer protector is the only structural part of protector.

In an embodiment of the present invention, the transducer protector is formed by the integrally molded part and the hydrophobic gas-permeable membrane, and optionally one or more sealing elements, with no further parts attached to transducer protector.

In an embodiment of the present invention, the hydrophobic gas-permeable membrane is bonded to a step portion of the transducer protector, the bonding area of the hydrophobic gas-permeable membrane being accessible from the second end of the fluid path. This simplifies manufacture of the transducer protector.

In an embodiment of the present invention, the step portion is provided within the fluid path or at the second end of the fluid path.

In an embodiment of the present invention, the inner diameter of the fluid path between its second end and the step portion is larger than the inner diameter of the fluid path at the step portion.

In an embodiment of the present invention, the inner diameter and/or the cross-section of the fluid path at its second end, and preferably between its second end and the hydrophobic gas-permeable membrane is larger than the outer diameter and/or outer shape of the hydrophobic gas-permeable membrane.

This simplifies the mounting of the hydrophobic gas-permeable membrane to the step portion, because the membrane and/or a bonding tool can be inserted into the transducer protector through the second end for manufacturing the transducer protector.

In an embodiment of the present invention, the second end of the fluid path is formed by a tubular element configured for sealingly connecting to the pressure transducer interface, an inner diameter of the tubular element being larger than the inner diameter of a tubular element forming the first connector. Preferably, the inner diameter of the tubular element forming the second end of the fluid path is at least 120 % of the inner diameter of a tubular element forming the first connector, preferably at least 150 %. These features are preferred for transducer protectors according to all aspects of the present invention.

In an embodiment of the present invention, the inner diameter and/or the inner cross-section of the step portion is larger than the inner diameter and/or the cross-section of the first connector and/or the pipe connected thereto. This provides an effective surface of the hydrophobic gas-permeable membrane that is larger than the cross-section of the first connector and/or the pipe connected thereto.

In an embodiment of the present invention, the transducer protector is formed by two integrally molded parts fixedly connected to each other, wherein preferably a first part comprises the first connector and the at least one bayonet connection element, and a second part comprises the second tubular element, wherein preferably the hydrophobic membrane is arranged between the first and the second part.

The present invention further comprises a pressure transducer interface configured for connecting to the second connector of a transducer protector as defined above, the pressure transducer interface comprising a fluid path coupled to a pressure transducer and a bayonet counter element, i.e. a bayonet mount, for engaging with a bayonet geometry of a bayonet element of the second connector. The pressure transducer interface provides the same advantages as described above for the transducer protector according to the first aspect. The pressure transducer interface may comprise the pressure transducer.

In an embodiment of the present invention, the pressure transducer interface comprises a sealing element for sealingly engaging a tubular section of the second connector of the transducer protector. The sealing element may contact a front surface of the tubular element and/or an inner or outer surface of the tubular element. The sealing element may be provided by a sealing ring or a conical section.

In an embodiment of the present invention, the sealing element engages with a front surface of the tubular section or a side surface of the tubular section.

In an embodiment of the present invention, the sealing element is formed by a cone section engaging with an inner cone section of the tubular section, in particular with a Luer cone section.

In an embodiment of the present invention, the bayonet counter element is formed by a bracket or a ring or connection plate, which preferably has at least one section with one or more notches and one or more abutments for engaging with the bayonet geometry of the bayonet connection element of the second connector, the bracket or a ring or connection plate freely extending between two connection sections where it is connected to a main surface of the pressure transducer interface. The bracket or a ring or connection plate may in particular be spaced apart from the main surface towards the transducer protector.

The brackets or a ring or connection plate allow a user to access from below the bracket or a ring or connection plate to easily clean/disinfect around the fluid path of the pressure transducer interface.

In an embodiment of the present invention, the main surface is an essentially flat surface from which the connection sections for the bracket or a ring or connection plate projects in a direction towards the transducer protector.

In a variant, the counter element is formed by an undercut of a recess provided in a main surface of the pressure transducer interface.

The pressure transducer interface and/or the transducer protector may have a feature to prevent the improper mounting, in particular to avoid a mounting in a position that is rotated by 90° around its axis.

In an embodiment of the present invention, the bayonet mount element comprises at least one axial channel and at least one circumferential support for engaging with an axial pin of the bayonet geometry of the second connector.

In an embodiment, the circumferential support is arranged adjacent to the axial channel to allow the pin to be arranged below the circumferential support, with the circumferential support comprising a recess to accommodate the pin in an engaged position.

In an embodiment, the axial channel is provided on an inner edge of a connection plate, and the circumferential support is provided by a lower surface of the bracket or a ring or connection plate.

The brackets or ring or connection plate may extend circumferentially around a tubular element of the pressure transducer interface. In an embodiment, the ring or connection plate may have a circular opening allowing insertion of a tubular element of the pressure transducer, with the axial channel or channels extending from the inner edge of the circular opening.

In an embodiment of the present invention, the pressure transducer interface comprises, in addition to the bayonet counter element, a threaded section configured for engaging with another type of transducer protector comprising a Luer connector.

Such a pressure transducer interface is compatible both with prior art transducer protectors and with the inventive transducer protector.

In an embodiment of the present invention, the bayonet mount element comprises at least one sealing element.

In an embodiment of the present invention, the sealing element is configured to sealingly engage with the second end of the fluid path of the transducer protector and in particular with a tubular element of the transducer protector forming the second end of the fluid path of the transducer protector.

In an embodiment of the present invention, the sealing element is a sealing ring arranged in a groove and/or configured to sealingly engage with the inner surface of the second tubular element of the second connector.

In an embodiment of the present invention, the pressure transducer interface comprises a third tubular element to be sealingly engaged with the second end of the fluid path of the transducer protector.

In an embodiment of the present invention, the sealing element is provided on an outer surface of the third tubular element to engage with the inner surface of a second tubular element of the second connector.

In an embodiment of the present invention, the sealing element is a sealing ring arranged in a groove provided on the outer surface of the third tubular element.

In an embodiment of the present invention, the third tubular element has an outer surface to engage with an inner surface of the second tubular element, wherein preferably, the outer surface is cone-shaped.

In an embodiment of the present invention, in an axial direction, a bayonet-geometry of the bayonet mount is provided between a first axial end and a second axial end of the outer surface of the third tubular element.

In an embodiment of the present invention, the bayonet mount comprises a spring element and/or an elastic sealing element.

In an embodiment of the present invention, the spring element and/or an elastic sealing element is configured to engage with the transducer protector to resiliently push the second connector in an axial direction away from the pressure transducer interface and thereby secure the bayonet connection.

In an embodiment of the present invention, the spring element and/or an elastic sealing element is configured to engage with a front surface of a tubular element forming the second end of the fluid path of the transducer protector and/or is arranged to sourround a third tubular element of the pressure transducer interface.

In a first aspect, the present invention further comprises a system comprising a transducer protector as described above and an adapter, the adapter comprising a second main body comprising a second fluid path, a third connector for releasably coupling a first end of the second fluid path to the fluid path of the transducer protector, and a fourth connector for releasably coupling a second end of the second fluid path to a pressure transducer interface. According to the first aspect, the third connector comprises a bayonet mount for connecting to the second connector of the transducer protector. The adapter can therefore provide a bayonet mount for connecting the inventive transducer protector to a pressure transducer interface, and therefore allows to use the present invention also on blood treatment machines not equipped with a pressure transducer interface of the present invention, but e.g. a standard pressure transducer interface.

In an embodiment of the first aspect, the fourth connector is of a different connector type than the third connector. In the context of the present invention, this means that the third connector could not be connected to the fourth connector.

In an embodiment of the first aspect, the fourth connector has a sealing cone and/or a screw element to engage with corresponding geometries of the pressure transducer interface.

In an embodiment of the first aspect, the fourth connector is configured as a Luer connector, in particular a female Luer connector.

In an embodiment of the first aspect, the fourth connector comprises a fourth tubular element forming the second end of the second fluid path, wherein preferably, the fourth tubular element has an inner surface configured as a sealing cone and a screw element arranged on its outer surface.

The third connector and in particular the bayonet mount may be configured in exactly the same way as described above with respect to the pressure transducer interface of the present invention. Further, all the features described in the following for the adapter of the first aspect may equally be applied to the pressure transducer interface of the present invention.

In an embodiment of the first aspect, the first end of the second fluid path of the adapter is configured as a third tubular element to be sealingly engaged with the second end of the first fluid path of the transducer protector.

In an embodiment of the first aspect, the adapter comprises a sealing element to sealingly engage with the second end of the first fluid path of the transducer protector and in particular with a second tubular element of the transducer protector forming the second end of the first fluid path of the transducer protector.

In an embodiment of the first aspect, the sealing element is provided on an outer surface of the third tubular element to engage with the inner surface of a second tubular element of the second connector.

In an embodiment of the first aspect, the sealing element is a sealing ring arranged in a groove provided on the outer surface of the third tubular element.

In an embodiment of the first aspect, the third tubular element has an outer surface to engage with an inner surface of the second tubular element, wherein preferably, the outer surface is cone-shaped.

In an embodiment of the first aspect, in an axial direction, a counter-geometry of the counter-element is provided between a first axial end and a second axial end of the outer surface of the third tubular element.

In a radial direction, the counter-element is provided on an outer side of the third tubular element with a free space provided between the counter-element and the third tubular element to accommodate the second tubular element and/or a bayonet connection element of the second connector.

In an embodiment of the first aspect, the counter-element has at least one bayonet counter-geometry to bayonet engage with a connection element of the second connector.

In an embodiment of the first aspect, the bayonet counter-geometry is arranged on an inner side of the counter-element to engage with a bayonet geometry arranged on an outer side of the connecting element.

In an embodiment of the first aspect, the bayonet counter-geometry comprises an axial channel and a circumferential support for engaging with an axial pin of the Bayonet geometry of the second connector, wherein preferably, the circumferential support is arranged adjacent to the axial channel to allow the pin to be arranged below the circumferential support, with the circumferential support comprising a recess to accommodate the pin in an engaged position.

In an embodiment of the first aspect, the third connector comprises a spring element to resiliently push the second connector in an axial direction away from the third connector and thereby secure the bayonet connection and in particular the pin in the recess.

In an embodiment of the first aspect, the spring element is configured to engage with a front surface of a second tubular element forming the second end of the fluid path of the transducer protector and/or is arranged to sourround a third tubular element of the adapter.

The spring element may be provided by an elastic sealing element.

In an embodiment of the first aspect, the third connector comprises at least two counter-elements to engage with corresponding connection elements of the second connector, wherein preferably, the counter-elements are arranged on opposite sides of an axis defined by the second fluid path at its first end.

In an embodiment of the first aspect, the at least two counter-elements are arranged on a wall extending circumferentially around the first end of the second fluid path, wherein preferably, the wall extends at a distance to a third tubular element of the third connector, the third tubular element forming the second fluid path at its first end, wherein preferably, the at least two counter-elements extend from a free end of the wall to an inside towards the second fluid path.

In an embodiment of the first aspect, the second fluid path extends along a single direction through the adapter.

In an embodiment of the first aspect, the adapter is integrally formed as a single element, in particular by injection molding.

In an embodiment of the first aspect, the adapter does not comprise a membrane, i.e. the second fluid path extends through the adapter without a barrier.

The present invention further comprises an adapter according to the first aspect. In particular, the adapter comprises a second main body comprising a second fluid path, a third connector for releasably coupling a first end of the second fluid path to the fluid path of the transducer protector, and a fourth connector for releasably coupling a second end of the second fluid path to a pressure transducer interface. According to the first aspect, the third connector comprises a bayonet mount for connecting to the second connector of the transducer protector.

The adapter is in particular configured as described above with respect to the inventive system.

In a second aspect, the present invention further comprises a system comprising a pressure transducer interface of the present invention as described above and an adapter, the adapter comprising a second main body comprising a second fluid path, a third connector for releasably coupling a first end of the second fluid path to the fluid path of a transducer protector, and a fourth connector for releasably coupling a second end of the second fluid path to the pressure transducer interface, wherein the fourth connector is a bayonet connector configured to connect to the bayonet mount of the pressure transducer interface.

The adapter of the second aspects can therefore be used for connecting a transducer protector to a pressure transducer interface of the present invention having a bayonet mount, and therefore allows to use on blood treatment machines of the present invention also transducer protectors not equipped with a bayonet connector of the present invention, but e.g. a standard connector.

In an embodiment of the second aspect, the fourth connector is of a different connector type than the third connector. In the context of the present invention, this means that the third connector could not be connected to the fourth connector.

In an embodiment of the second aspect, the third connector has a sealing cone and/or a screw element to engage with corresponding geometries of the second connector.

In an embodiment of the second aspect, the third connector is configured as Luer connector, in particular as a male Luer connector, in particular as a Luer cone connector.

In an embodiment of the second aspect, the third connector comprises a male sealing cone forming the first end of the second fluid path and a third tubular element extending around the male sealing cone and comprising a screw element arranged on its inner surface.

The fourth connector and in particular the bayonet connection element of the fourth connector may be configured in exactly the same way as described above with respect to the transducer protector of the present invention. Further, all the features described in the following for the adapter of the second aspect may equally be applied to the transducer protector of the present invention.

In an embodiment of the second aspect, the fourth connector comprises at least one connection element connected to a main body of the adapter and having a free end comprising a connection geometry for bayonet engaging the counter element of the pressure transducer interface.

In an embodiment of the second aspect, the connection element extends from a connection point where it is connected to the main body of the adapter in a direction substantially along of the second fluid path of the adapter towards the pressure transducer interface.

In an embodiment of the second aspect, the second end of the second fluid path is configured as a fourth tubular element to be sealingly engaged with a fluid path of the pressure transducer interface.

In an embodiment of the second aspect, the at least one connection element of the fourth connector extends from a connection point with the main body of the adapter towards the pressure transducer interface on an outside and further preferably with a distance to the fourth tubular element.

In an embodiment of the second aspect, the connection geometry of the at least one connection element of the fourth connector is arranged on an outer side of the connection element with respect to a radial direction.

In an embodiment of the second aspect, the connection geometry comprises a bayonet pin extending in a radial direction.

In an embodiment of the second aspect, the fourth connector comprises at least two separate connection elements, the connection elements being preferably arranged on opposite sides of an axis defined by the second fluid path at its second end.

In an embodiment of the second aspect, the fourth connector comprises at least one bayonet connection element, wherein the bayonet connection element is preferably connected to a main body of the transducer protector and has a free end comprising a bayonet geometry for engaging a counter element of the pressure transducer interface, in particular at least one pin extending in a radial direction.

In an embodiment of the second aspect, the at least one bayonet connection element extends from a connection point where it is connected to the main body of the adapter in a direction along of the second fluid path towards the pressure transducer interface.

In an embodiment of the second aspect, the Bayonet geometry comprises an axial pin arranged on the connection element.

In an embodiment of the second aspect, the fourth connector comprises an abutting surface configured to engage with a spring element arranged on the pressure transducer interface, wherein preferably, the abutting surface is formed by a fourth tubular element forming the second end of the second fluid path.

In an embodiment of the second aspect, the second fluid path extends along a single direction through the adapter.

In an embodiment of the second aspect, the adapter is integrally formed as a single element, in particular by injection molding.

In an embodiment of the second aspect, the adapter does not comprise a membrane, i.e. the second fluid path extends through the adapter without a barrier.

The present invention further comprises an adapter according to the second aspect. In particular, the adapter comprises a second main body comprising a second fluid path, a third connector for releasably coupling a first end of the second fluid path to the fluid path of the transducer protector, and a fourth connector for releasably coupling a second end of the second fluid path to a pressure transducer interface. According to the second aspect, the fourth connector is a bayonet connector for connecting to a bayonet mount of the pressure transducer interface.

The adapter is in particular configured as described above with respect to the inventive system according to the second aspect.

The present invention further comprises a tubing system comprising a transducer protector as defined herein and at least one component of an extracorporeal blood circuit.

In particular, the component of the extracorporeal blood circuit is connected to the first connector via a tube, preferably glued to or inserted into a section of the first connector.

In an embodiment, the component of the extracorporeal blood circuit is connected to the first connector via a third connector. The third connector may, e.g., be a male or female Luer connector, a bayonet connector or screw-type connector.

In an embodiment, the component of the extracorporeal blood circuit is connected to the first connector via a third connector. The third connector may, e.g., be a male or female Luer connector. The Luer connector may be part of a transducer protector and comprising a hydrophobic gas-permeable membrane. In this case, the bayonet transducer protector of the present invention provides a second hydrophobic gas-permeable membrane for additional safety in case of a rupture of the first membrane.

In a further embodiment, the component of the extracorporeal blood circuit is connected to the first connector via a third connector. The third connector may, e.g., be a male or female Luer connector, a bayonet connector or screw-type connector. The third connector may be part of a transducer protector comprising a hydrophobic gas-permeable membrane. The bayonet transducer protector of the present invention does not comprise a hydrophobic gas-permeable membrane. Here, the transducer protector serves as an adapter from male or female Luer connector or a bayonet connector or screw-type connector to bayonet connector.

Preferably, the tube is a tube leading from a venous chamber of the extracorporeal blood circuit for conveying air across the hydrophobic gas-permeable membrane.

The component of the extracorporeal blood circuit may in particular be a venous chamber.

The tubing system may comprise further components of an extracorporeal blood circuit, such as a venous and/or an arterial line or connection lines connectable to a venous and/or an arterial line.

The present invention further comprises a system comprising an adapter according to the first or the second aspect and at least one out of an extracorporeal blood circuit and a transducer protector, in particular a transducer protector of the present invention as described above.

The present invention further comprises a system comprising a transducer protector as defined herein and a pressure transducer interface as defined herein.

The present invention further comprises a blood treatment machine, in particular a dialysis machine, comprising a pressure transducer interface as defined herein.

The present invention further comprises a blood treatment machine, in particular a dialysis machine, comprising a system according to the first or the second aspect.

In the blood treatment machines of the present invention, the pressure transducer interface may be provided on a casing of the blood treatment machine. The main surface of the pressure transducer interface may be provided by an outer surface of the casing.

The present invention further comprises a blood treatment machine, in particular a dialysis machine, comprising a pressure transducer interface and a transducer protector as defined herein. The blood treatment machine may further comprise an extracorporeal blood circuit.

The present invention further comprises the use of a transducer protector according to the present invention for connecting a pipe of an extracorporeal circuit to a pressure transducer by bayonet connecting a bayonet element of the second connector of the transducer protector to a bayonet counter element of a pressure transducer interface, in particular to a pressure transducer interface of a blood treatment machine, in particular of a blood treatment machine as described above.

The present invention further comprises the use of an adapter and/or a system according to the first or the second aspect for connecting a pressure transducer to a pressure transducer interface of a blood treatment machine, in particular of a blood treatment machine as defined above.

In a further independent aspect, the present invention comprises a method for manufacturing a transducer protector of the present invention; the method comprising the step of:
- integrally molding the main body of the protector comprising the entire fluid path and preferably both the first and the second connector,
   and/or
- bonding the hydrophobic gas-permeable membrane to a step portion of the transducer protector by accessing the bonding area of the hydrophobic gas-permeable membrane from and preferably through the second end of the fluid path, the step portion being preferably provided within the fluid path or at the second end of the fluid path.

In the context of the present invention, the terms axial and radial are always defined with respect to an axis provided by the respective fluid path and/or tubular element.

The present invention is now described in further detail with respect to drawings and embodiments.

In the drawings, the figures show the following:
- Fig. 1: the first embodiment of a transducer protector, coupled to a first embodiment of a pressure transducer interface according to the present invention, in sectional view,
- Fig. 2: the first embodiment in a sectional view when seen from the first side,
- Fig. 3: the first embodiment in a perspective view when seen from the first side,
- Fig. 4: the first embodiment of a transducer protector, inserted into a first embodiment of a pressure transducer interface according to the present invention, in a perspective view,
- Fig. 5: the first embodiment of a transducer protector connected to a second embodiment of a pressure transducer interface according to the present invention, in a perspective view,
- Fig. 6: a transducer protector adapter according to the second aspect coupled to the pressure transducer interface according to the present invention, in a sectional view, forming a system according to the first aspect,
- Fig. 7: the transducer protector adapter according to the second aspect coupled to the pressure transducer interface according to the present invention, in a perspective view, forming a system according to the first aspect,
- Fig. 8: a transducer protector adapter according to the first aspect coupling a transducer protector according to the present invention to a standard pressure transducer interface a in a sectional view, the adapter and the transducer protector forming a system according to the first aspect,
- Fig. 9: an embodiment of a tubing set comprising a component of an extracorporeal blood circuit and the inventive transducer protector.

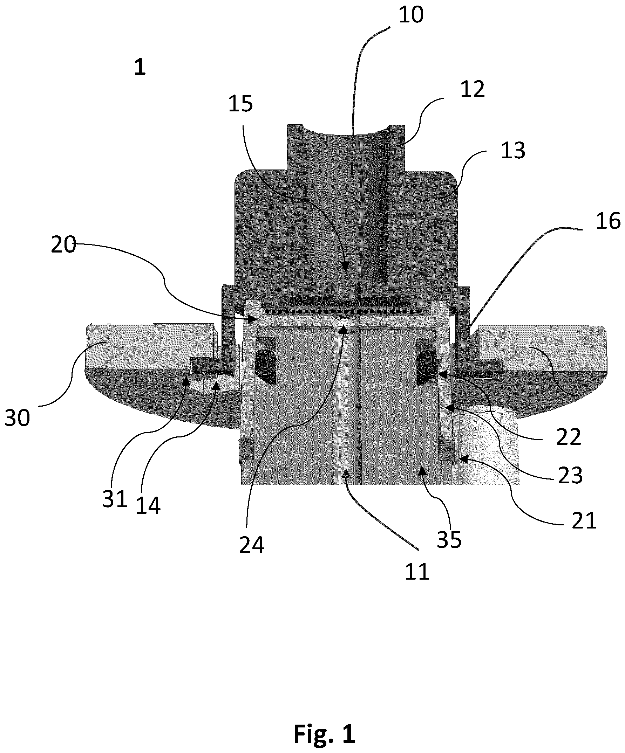

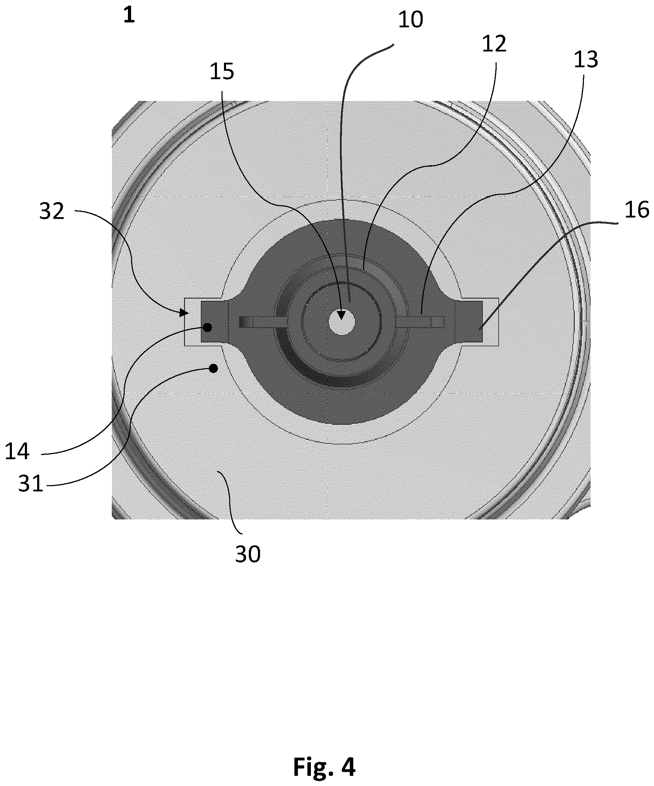

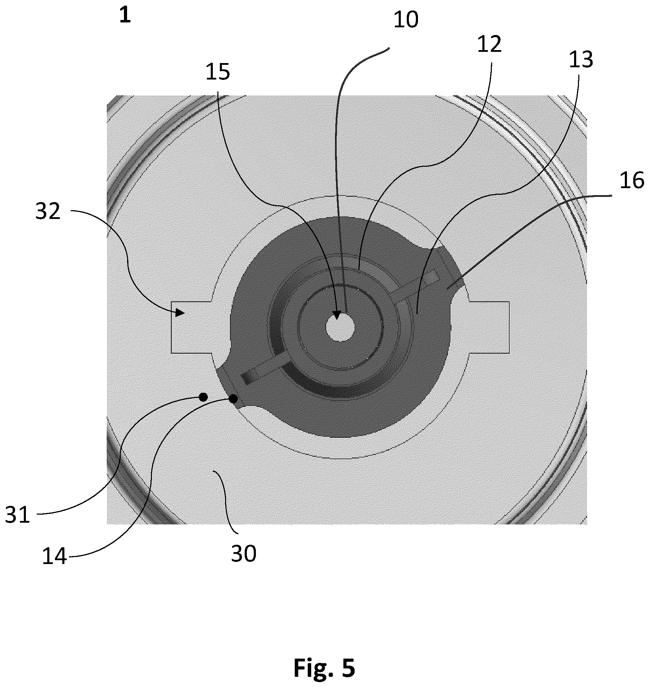

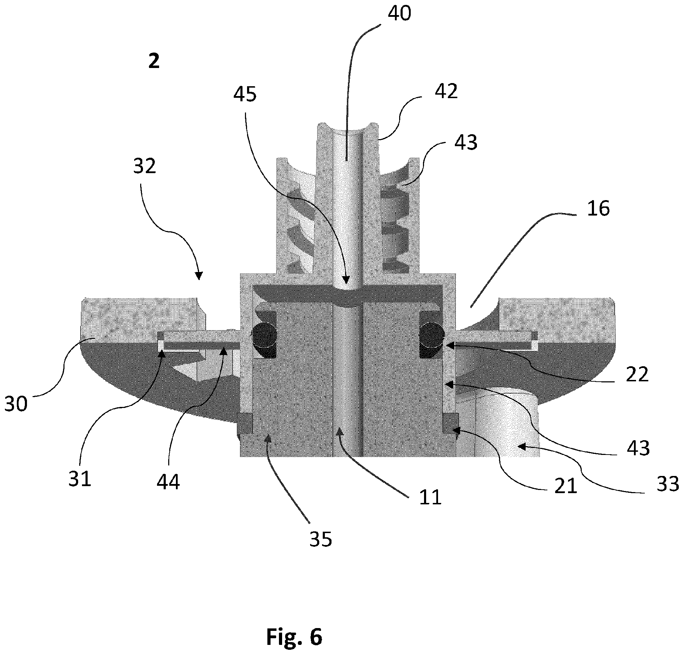

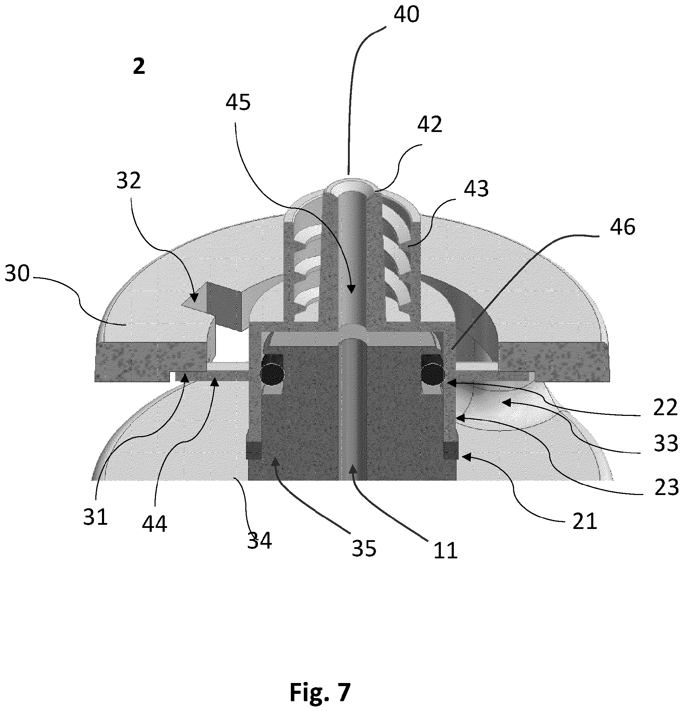

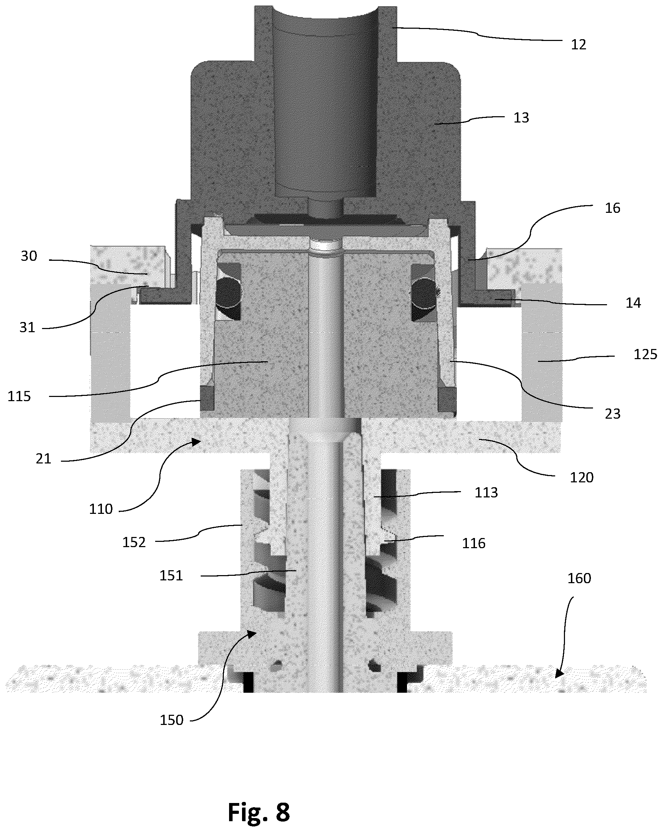

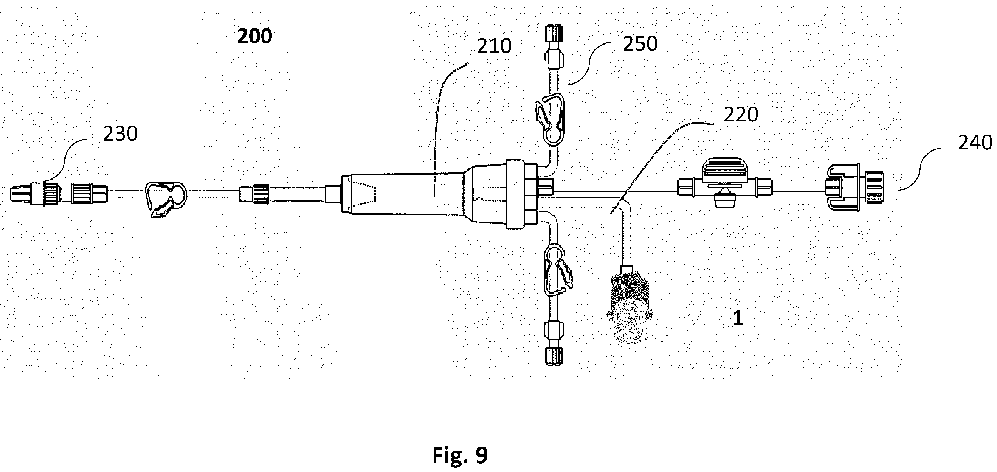

Fig. 1 - 5 show a first embodiment of a transducer protector according to the present invention. A transducer protector adapter and system according to the second aspect is shown in Fig. 6 and 7. A transducer protector adapter and system according to the first aspect is shown in Fig. 8. Fig. 9 shows an embodiment of a tubing set comprising an extracorporeal blood circuit and the inventive transducer protector. If not otherwise stated, the features and the configuration of the transducer protector described in the following applies to all the embodiments.

Further, the first embodiment of a transducer protector, as well as most of the other embodiments, embody all the aspects of the present invention in combination. However, the features described with respect to the respective aspects could also be used independently from the other aspects.

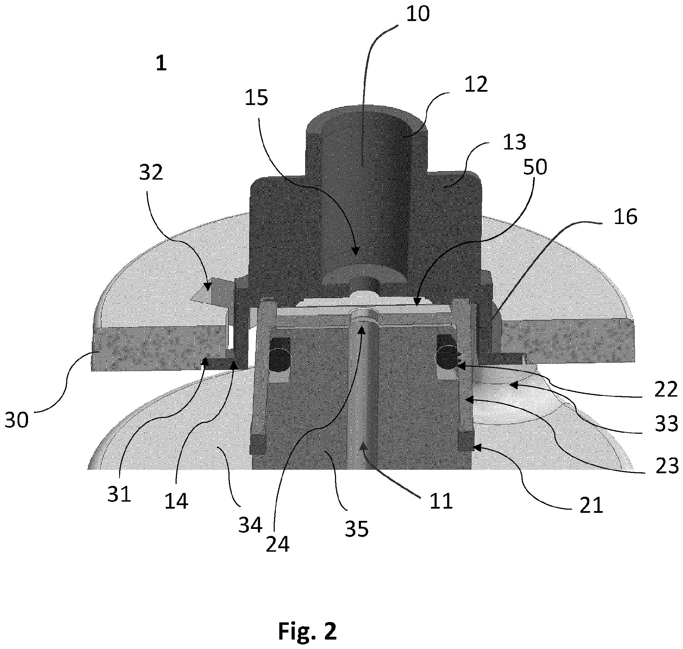

According to the present invention, the transducer protector 1 comprises a main body 12 comprising a fluid path 15 schematically shown by an arrow in Fig. 1. The fluid path 15 passes through the main body of the transducer protector from a first end 10 to a second end 11. Further, a hydrophobic gas-permeable membrane 50 shown in Fig. 2 is arranged in the fluid path 15 for separating the first end of the fluid path from the second end.

The hydrophobic gas-permeable membrane is in particular an air-permeable hydrophobic membrane. It therefore provides an air-permeable and therefore pressure-permeable connection through the connector but is impermeable to liquids and therefore protects the second end 11 from blood entering through the first end 10.

The hydrophobic gas-permeable membrane may comprise a polytetrafluoroethylene (ePTFE) polymer.

In one embodiment the membrane is made of a PFAS-free polymeric material. The PFAS-free polymeric material may be selected from polyurethane (PU), polyethylene (PE), polypropylene (PP), polyester, polyethylene terephthalate (PET), polycarbonate (PC), polyimide (PI), polyethylene naphthalate (PEN), polyethersulfone (PES), polyether ether ketone (PEEK), and combinations thereof. The membrane may comprise one or more reinforcement layers and/or one or more coating layers.

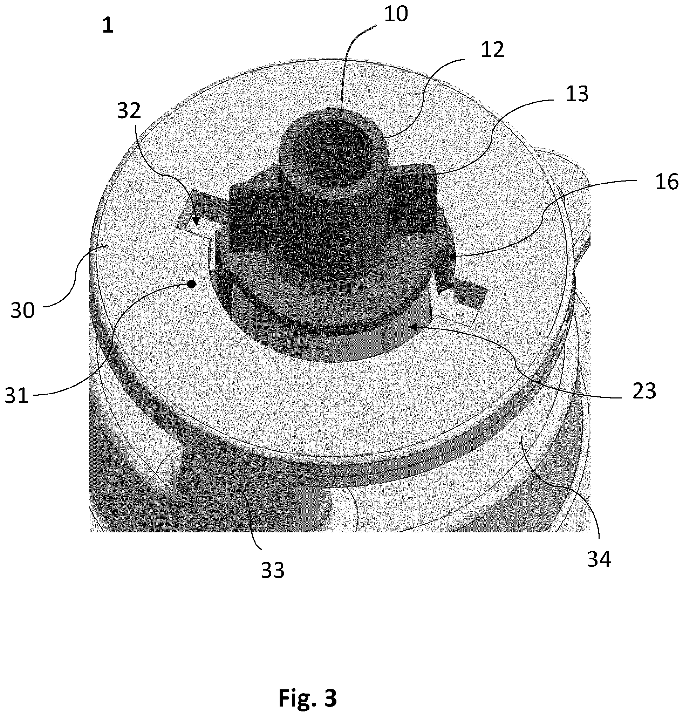

The transducer protector 1 comprises a first connector 10 for fluidly coupling the first end 12 of the fluid path to a pipe of an extracorporeal circuit. In particular, as shown in Fig. 2 and 3, the first connector may be provided by a tubular element 12, into which the distal end of the pipe of the extracorporeal circuit may be inserted. The end of the pipe may be bonded to the first connector and in particular to the inside of the tubular element 12 by an adhesive.

In the embodiment, a handling section of the transducer protector is arranged on a first tubular element 12. The handling section comprises two handling elements 13 extending radially from the first tubular element 12. The handling elements 13 are connected axially to a ring element of the transducer protector.

The transducer protector further comprises a second connector 20 for fluidly coupling the second end 24 of the fluid path to a pressure transducer interface, such as one of the pressure transducer interfaces shown in Fig. 1 - 5. If not indicated otherwise, the second connector 20 comprises a second tubular element 23 for sealingly connecting to a fluid path of the pressure transducer interface. If not indicated otherwise, the second tubular element 23 has a larger diameter than the first tubular element 10 forming the first connector and/or a larger diameter than the membrane 50.

According to the first aspect of the present invention, the second connector 20 of the present invention is configured as a bayonet connector. In the embodiment shown, the bayonet connection between the second connector and the pressure transducer interface is engaged by an axial and rotational movement of the transducer protector.

Therefore, for closing the bayonet connection, no rotating movement with increasing torque is needed. In contrast to the prior art solutions using a Luer connection, fixation is ensured by a pushing and turning movement. This provides an improved usability and ensures gas-tightness of the connection.

Further, due to the bayonet connection, a proper connection is ensured by tactile and audible feedback. In particular, the bayonet connection will provide a click sound and an abrupt jump in the counter pressure against an axial movement when the bayonet connection is engaged.

Fig. 6 and 7 show the transducer protector adapter 2 according to the second aspect coupled to the pressure transducer interface according to the present invention, in a sectional and a perspective view. According to the present invention, the transducer protector adapter 2 comprises a main body 42 comprising a fluid path 45 schematically shown by an arrow in Figs. 6 and 7. The fluid path 45 passes through the main body of the transducer protector adapter from a first end 40 to a second end 11 of the transducer interface 35. Optionally, a hydrophobic gas-permeable membrane 50 not shown in Fig. 6 is arranged in the fluid path 45 for separating the first end of the fluid path from the second end.

The optional hydrophobic gas-permeable membrane 50 is in particular an air-permeable hydrophobic membrane. It therefore provides an air-permeable and therefore pressure-permeable connection through the connector but is impermeable to liquids and therefore protects the second end 11 from blood entering through the first end 10.

The transducer protector adapter 2 comprises a first connector 40 for fluidly coupling the first end 42 of the fluid path to a pipe 42 of a Luer connector. In particular, as shown in Fig. 6 and 7, the first connector may be provided by a threaded screw element 43, into which the distal end of the pipe of the extracorporeal circuit may be inserted. The end of the pipe may comprise a transducer protector with a male or female Luer connector, a bayonet connector or screw-type connector.

The transducer protector adapter further comprises a second connector for fluidly coupling the second end 43 of the fluid path to a pressure transducer interface, such as one of the pressure transducer interfaces shown in Fig. 1 - 5. If not indicated otherwise, the second connector comprises a second tubular element 43 for sealingly connecting to a fluid path of the pressure transducer interface. If not indicated otherwise, the second tubular element 43 has a larger diameter than the first tubular element 42 forming the first connector and/or a larger diameter than the optional membrane 50.

In a preferred embodiment, the transducer protector adapter is made in one piece by injection molding and does not comprise a gas-permeable membrane.

In all the embodiments shown, the fluid path defines a single axis through the connector, with the first tubular element 20 and the second tubular element 37 being arranged co-linear with respect to each other.

In the embodiment shown in Fig. 1 - 4 and 5-6, the transducer protector (adapter) has the bayonet connection elements 14 and 16 arranged on an outer side of the body 12 or 42, respectively, and therefore engages with the counter elements of the pressure transducer interface on its outside. The bayonet connection elements 14 and 16 extend towards the pressure transducer interface comprise pins extending in a radial direction.

For disengaging the bayonet connection, the transducer protector is pushed towards the pressure transducer interface and turned in the opposite direction of the rotation used during the engagement of the transducer protector.

Apart from a pressure transducer, the present invention also provides a novel pressure transducer interface configured for engaging with the inventive transducer protector.

In these embodiments, the interface is configured to engage with the transducer protector. The counter element 30 or bayonet mount of the transducer interface therefore is provided with a bayonet geometry and in particular one or more notches 32 and recesses 31 on its inner side, engaging with the bayonet geometry of the transducer protector provided on the outside of the bayonet connection elements of the first embodiment shown in Fig. 1 - 5. The notches may in particular provide an axial channel for inserting the bayonet pins of the bayonet elements. The recesses are provided on the lower surface of the bayonet counter geometry 30.

The sealing between the transducer protector and the pressure transducer interface is provided, in all the embodiments, by a sealing element 22 arranged on the side of the pressure transducer interface. The sealing element 22 is configured to sealingly engage with the second tubular section 23 of the transducer protector. In particular, the sealing element 22 is provided by an O-ring made from an elastomeric material.

In the embodiments shown in Fig. 1 and 2, and Fig. 6 and 7 the elastic sealing element 22 engages with an inner side of the tubular element 23, 43.

The pressure transducer interface further comprises a spring element 21 that resiliently urges the transducer protector away from the pressure transducer interface to secure the bayonet connection by pressing the bayonet pins 14 into the recesses 31.

The spring element may be provided as an elastic sealing element.

In the embodiments shown in Fig. 1 and 2, and Fig. 6 and 7 the spring element / elastic sealing element 21 engages with a front surface of the tubular element 23. In Fig. 6 and 7, the spring element / sealing element 21 engages with a front surface of the tubular element 43 of the adapter.

In one embodiment, the sealing element 22 has a rectangular cross-section and is provided in a groove facing the transducer protector.

The sealing elements 21 and 22 provide gas-tighness to the connection and ensure a reliable pressure measurement by the pressure transducer.

The sealing element 21 may be made of flexible, compressible polymer material and may provide counter-pressure to the tubular section 23 of the transducer protector 1 to hold the bayonet connector elements 14 in the notches 31 of the pressure transducer interface 30.

The sealing element 21 may be made of flexible, compressible polymer material and may provide counter-pressure to the tubular section 43 of the transducer protector adapter 2 to hold the bayonet connector elements 44 in the notches 31 of the pressure transducer interface 30.

The bayonet counter geometry is provided on a connection plate or bracket extending at a distance to the surface 34 of the pressure transducer interface, and is connected thereto by connection webs 33.

The brackets and/or connection plate therefore can be accessed by a user from below to easily clean and disinfect around the fluid connection provided between the brackets.

The bonding of the hydrophobic membrane is done by heat bonding, ultrasonic welding or gluing. The preferred method for bonding the membrane to the sealing layer is ultrasonic welding.

In all the embodiments, due to the bayonet connection, the present invention provides a decisively easier handling. Further, for those embodiments where the main body of the transducer protector is provided as a single piece to be manufactured by injection molding, or the membrane is bonded to the main body from the second side of the fluid path, the manufacture is simplified with respect to the two-shell-design of the prior art solution.

Fig. 8 shows an embodiment of an adapter 110 according to the first aspect, which connects a transducer protector according to the present invention to a pressure transducer interface 150. Therefore, the bayonet mount as described above is provided on the adapter.

Specifically, the adapter 110 comprises a second main body comprising a second fluid path 111, a third connector for releasably coupling a first end of the second fluid path 111 to the first fluid path of the transducer protector, and a fourth connector for releasably coupling a second end of the second fluid path 111 to the pressure transducer interface 150. In the embodiment, the second fluid path 111 extends axially through the adapter.

According to the first aspect, the third connector is a bayonet mount for connecting to the bayonet connector of the transducer protector.

Therefore, all the configurations of the pressure transducer interfaces described above may be provided on the third connector of the adapter rather than on the pressure transducer interface.

In the embodiment, the first end of the second fluid path 111 of the adapter 110 is configured as a third tubular element 112 to be sealingly engaged with the second end of the first fluid path of the transducer protector.

In the embodiment, the counter element 30 of the transducer interface are provided with a bayonet counter geometry as described above.

In the embodiment, the bayonet counter geometry is provided on the inner side of the counter element 30, engaging with the bayonet geometry and in particular the radial pins of the transducer protector provided on the outside of the bayonet elements 14, 16.

The sealing between the transducer protector and the adapter 110 is provided by a sealing element arranged on the side of the adapter. The sealing element is configured to sealingly engage with the second tubular section 23 of the transducer protector. In particular, the sealing element is provided by an O-ring made from an elastomeric material.

In the embodiment, the sealing element engages with an inner side surface of the tubular element 23. In an alternative configuration, it could also engage with an outer side surface of the tubular element 23. In Fig. 8, the sealing element is provided in a groove provided on an outer surface of the third tubular element 115.

In the embodiment, the third tubular element 115 has an outer surface to engage with an inner surface of the second tubular element 23, with the outer surface being cone-shaped. The tubular element as well as the cone shape will help to guide the second tubular element on the third tubular element in order to avoid a misalingement.

In the embodiment, in an axial direction, a counter-geometry of the counter-element 30 is provided between a first axial end and a second axial end of the outer surface of the third tubular element 115.

In a radial direction, the counter-element 30 is provided on an outer side of the third tubular element 115 with a free space provided between the counter-element 30 and the third tubular element 115 to accommodate the second tubular element 23 and the bayonet connection elements 14, 16 of the second connector.

As shown in Fig. 8, the third connector comprises two counter-geometries to engage with corresponding connection elements of the second connector, the counter elements 120 being arranged on opposite sides of an axis defined by the second fluid path 111 at its first end.

The adapter 110 may comprise an outer wall or connection elements 125, with the counter-element 30 arranged on a free end outer wall or connection elements 125. The outer wall or connection elements 125 extend at a distance to the third tubular element 115 and is connected to the third tubular element 115 by a bottom wall 120, such that the outer wall or connection elements 125 and the bottom wall 120 together have a cup shape with the third tubular element 115 extending from the bottom wall towards the open side of the cup shape.

In an embodiment of the present invention, the wall and/or the bottom wall are formed as closed surfaces. Alternatively, the wall and/or the bottom wall may have openings and/or may be provided by web elements.

In the embodiment shown in Fig. 8, fourth connector of the adapter is configured to sealingly engage the pressure transducer interface 150. The pressure transducer interface 150 is a different connector type than the pressure transducer interfaces according to the first aspect described so far. Therefore, the fourth connector is of a different connector type than the third connector.

The pressure transducer interface 150 and the fourth connector may be any type of connector, and in particular may be a standard connector as used in the art. The adapter 110 thereby allows to engage the transducer protector having a snap-fit connector to a pressure transducer interface 150 having a connector type to which the snap-fit connector of the transducer protector could not be connected.

In an embodiment of the present invention, pressure transducer interface 150 and the fourth connector are Luer type connectors. In particular, the fourth connector is configured as a female Luer connector and the pressure transducer interface 150 is configured as a male Luer connector.

In the embodiment, the fourth connector has a sealing cone, which is in particular provided on the inside of the fourth tubular element 113 forming the second end of the second fluid path 111, as well and a screw element 116 which in particular may be provided on the outer surface of the fourth tubular element 113 to engage with corresponding geometries of the pressure transducer interface 150.

The pressure transducer interface 150 comprises a male cone 151, and a tubular element 152 arranged to surround the male cone 151 and having screw threads provided on its inner side.

In the embodiment, the second fluid path 111 extends along a single direction through the adapter 110. Further, the second fluid path 111 is not provided with a membrane, but leads through the adapter without any barrier.

In the embodiment, the adapter is integrally formed as a single element, in particular by injection molding.

The pressure transducer interface 150 is arranged an outer surface 160 of a blood treatment machine, and in particular integrated in the casing of the blood treatment machine. The blood treatment machine is in particular a dialysis machine.

A pressure transducer is arranged inside the blood treatment machine and fluidly connected to the pressure transducer interface 150.

Fig. 9 shows a tubing set 200 comprising a transducer protector 1 connected by line 220 to a component of an extracorporeal blood tubing set. In the embodiment, the component to which the transducer protector 1 is connected by line 220 is a venous air trap chamber 210. Connected to chamber 210 are connections lines 230 and 240 for connection to a venous line and an arterial line, as well as one or more further connection lines 250.

The end of line 220 is inserted and glued into connector 10 of the transducer protector 1. The distal ends of connection lines 230, 240 and 250 are provided with connection elements for providing a releasable connection to further lines or components of the extracorporeal blood circuit, such as Luer-lock connectors.

## Claims

1. A transducer protector for an extracorporeal circuit, comprising:
a main body comprising a fluid path;
a first connector for fluidly coupling a first end of the fluid path to a pipe of the extracorporeal circuit;
a second connector for fluidly coupling a second end of the fluid path to a pressure transducer interface; and
a hydrophobic membrane arranged in the fluid path for separating the first end of the fluid path from the second end;
**characterized in that**
the second connector is a bayonet connector.

2. The transducer protector according to claim 1, wherein the second connector comprises at least one bayonet connection element, wherein the bayonet connection element is preferably connected to a main body of the transducer protector and has a free end comprising a bayonet geometry for engaging a counter element of the pressure transducer interface, wherein the bayonet geometry in particular comprises at least one pin extending in a radial direction,
wherein further preferably, the at least one bayonet connection element extends from a connection point where it is connected to the main body of the transducer protector in a direction along of the fluid path of the transducer protector towards the pressure transducer interface.

3. The transducer protector according to one of the preceding claims, wherein the second connector comprises at least two separate bayonet connection elements and/or bayonet connection geometries, in particular at least two pins extending in a radial direction, the bayonet elements and/or bayonet connection geometries being preferably arranged on opposite sides of an axis defined by the fluid path at its second end,
and/or
wherein the bayonet connection provided by the second connector is releasable by pushing the transducer protector in a direction towards the transducer interface and turning the transducer protector,
and/or
wherein the second end of the fluid path is configured as a tubular element to be sealingly engaged with a fluid path of the pressure transducer interface, wherein preferably a bayonet connection element or bayonet connection elements of the second connector extend from a connection point with the main body of the transducer protector towards the pressure transducer interface on an outside the tubular element.

4. The transducer protector according to one of the preceding claims,
wherein the transducer protector comprises an integrally molded part comprising the entire fluid path and preferably both the first and the second connector, and/or the hydrophobic membrane is bonded to a step portion of the transducer protector, the bonding area of the hydrophobic membrane being accessible from the second end of the fluid path, the step portion being preferably provided within the fluid path or at the second end of the fluid path, wherein preferably the inner diameter and/or the cross-section of the fluid path between its second end and the hydrophobic membrane is larger than the outer diameter and/or outer shape of the hydrophobic membrane, and/or wherein the inner diameter and/or the inner cross-section of the step portion is larger than the inner diameter and/or the cross-section of the first connector and/or the pipe connected thereto
or
wherein the transducer protector is formed by two integrally molded parts fixedly connected to each other, wherein preferably a first part comprises the first connector and the at least one bayonet connection element, and a second part comprises the second tubular element, wherein preferably the hydrophobic membrane is arranged between the first and the second part.

5. A pressure transducer interface configured for connecting to the second connector of a transducer protector as defined by one of the preceding claims, the pressure transducer interface comprising a fluid path coupled to a pressure transducer and a bayonet mount element for engaging with a bayonet geometry of a bayonet connecting element of the second connector.

6. The pressure transducer interface according to claim 5,
wherein the bayonet mount element is formed by a connection plate freely extending between two connection sections where it is connected to a main surface of the pressure transducer interface, wherein preferably, the main surface is an essentially flat surface from which the connection plate is spaced apart in a direction towards the transducer protector,
and/or
wherein the bayonet mount element comprises at least one axial channel and at least one circumferential support for engaging with an axial pin of the Bayonet geometry of the second connector, wherein preferably, the circumferential support is arranged adjacent to the axial channel to allow the pin to be arranged below the circumferential support, with the circumferential support comprising a recess to accommodate the pin in an engaged position, wherein preferably the axial channel is provided on an inner edge of a connection plate, and the circumferential support is provided by a lower surface of the connection plate, and/or
wherein the pressure transducer interface comprises, in addition to the bayonet counter element, a threaded section configured for engaging with another type of transducer protector comprising a Luer connector.

7. The transducer interface according to claim 5 or 6, wherein the bayonet mount element comprises at least one sealing element,
wherein preferably, the sealing element is configured to sealingly engage with the second end of the fluid path of the transducer protector and in particular with a tubular element of the transducer protector forming the second end of the fluid path of the transducer protector,
wherein preferably, the sealing element is a sealing ring arranged in a groove and/or configured to sealingly engage with the inner surface of the second tubular element of the second connector.

8. The transducer interface according to any one of claims 5 to 7, wherein the pressure transducer interface comprises a third tubular element to be sealingly engaged with the second end of the fluid path of the transducer protector, wherein preferably, the sealing element is provided on an outer surface of the third tubular element to engage with the inner surface of a second tubular element of the second connector,
wherein preferably, the sealing element is a sealing ring arranged in a groove provided on the outer surface of the third tubular element,
and/or
wherein preferably, the third tubular element has an outer surface to engage with an inner surface of the second tubular element, wherein preferably, the outer surface is cone-shaped,
and/or
wherein preferably, in an axial direction, a bayonet-geometry of the bayonet mount is provided between a first axial end and a second axial end of the outer surface of the third tubular element.

9. The transducer interface according to any one of claims 5 to 8,
wherein the bayonet mount comprises a spring element and/or an elastic sealing element,
wherein preferably, the spring element and/or an elastic sealing element is configured to engage with the transducer protector to resiliently push the second connector in an axial direction away from the pressure transducer interface and thereby secure the bayonet connection,
and/or wherein preferably, the spring element and/or an elastic sealing element is configured to engage with a front surface of a tubular element forming the second end of the fluid path of the transducer protector and/or is arranged to sourround a third tubular element of the pressure transducer interface.

10. A system comprising a transducer protector according to one of claims 1 to 4 and an adapter, the adapter comprising a second main body comprising a second fluid path, a third connector for releasably coupling a first end of the second fluid path to the fluid path of the transducer protector, and a fourth connector for releasably coupling a second end of the second fluid path to a pressure transducer interface, wherein the third connector comprises a bayonet mount for connecting to the second connector of the transducer protector, wherein preferably, the fourth connector is of a different connector type than the third connector.

11. A system comprising a pressure transducer interface according to any one of claims 5 to 9 and an adapter, the adapter comprising a second main body comprising a second fluid path, a third connector for releasably coupling a first end of the second fluid path to the fluid path of a transducer protector, and a fourth connector for releasably coupling a second end of the second fluid path to the pressure transducer interface, wherein the fourth connector is a bayonet connector configured to connect to the bayonet mount of the pressure transducer interface, wherein preferably, the third connector is of a different connector type than the fourth connector.

12. A connector of the system of claim 10 or 11.

13. A system comprising a transducer protector according to one of claims 1 to 4 and at least one out of an extracorporeal blood circuit and a pressure transducer interface according to any one of claims 7 to 12, or an adapter according to claim 12 and at least one out of an extracorporeal blood circuit and a transducer protector, in particular a transducer protector according to one of claims 1 to 4.

14. A blood treatment machine, in particular a dialysis machine, comprising a pressure transducer interface according to any one of claims 5 to 9 and/or a system according to one of claim 10, 11 and 13.

15. Use of a transducer protector according to one of claims 1 to 4 for connecting a pipe of an extracorporeal circuit to a pressure transducer by bayonet connecting a bayonet element of the second connector of the transducer protector to a bayonet mount of a pressure transducer interface, in particular of a pressure transducer interface of a blood treatment machine, in particular of a blood treatment machine according to claim 14, and/or
use of an adapter according to claim 12 for connecting a pressure transducer to a pressure transducer interface of a blood treatment machine, in particular of a blood treatment machine according to claim 14.
